# EUROPEAN PATENT APPLICATION

(11) **EP 4 276 740 A1**
(43) Date of publication of application: **15.11.2023**
(21) Application number: 21917646.8
(22) Date of filing: 15.12.2021
(51) Int. Cl.: G06T 7/00, G06T 7/60, G01N 33/36

(54) **ANALYSIS METHOD FOR IMAGES OF FIBER-CONTAINING PRODUCTS AND PROGRAM AND ANALYSIS DEVICE THEREFOR**

(30) Priority: 06.01.2021 JP 2021000899
(71) Applicant: TOYOBO CO., LTD., Osaka-shi Osaka 5300001 (JP)
(72) Inventor: ISHIHARA, Ryoichi, Otsu-shi, Shiga 520-0292 (JP); UCHIDA, Yoshihiro, Otsu-shi, Shiga 520-0292 (JP); FURUICHI, Kenji, Otsu-shi, Shiga 520-0292 (JP)
(74) Representative: Müller-Boré & Partner Patentanwälte PartG mbB
(86) International application number: PCT/JP2021/046269
(87) International publication number: WO 2022/149425

(57) **Abstract**

A fiber structure is estimated from an image of a product including a fiber. A method for analyzing an image of a product including a fiber includes: a step (STEP 1) of acquiring a three-dimensional image (310) of the product including the fiber; a step (STEP 2) of converting the fiber in the three-dimensional image (310) into a set (320) of particles; and a step (STEP 3, 4) of estimating a structure of the fiber based on a positional relationship between particles included in the set (320) of particles.

## Description

### TECHNICAL FIELD

The present disclosure relates to image analysis, and more particularly, to image analysis including a fiber.

### BACKGROUND ART

Analysis of a structure of a product including the fiber is required in order to improve quality of the product including the fiber such as a network structure having a random loop structure, paper, a nonwoven fabric, or felt. The product including the fiber has a structure in which a plurality of fibers are intricately entangled, and for example, the analysis of the structure of the product including the fiber includes the analysis of a shape of each fiber itself such as a thickness and a length and the analysis of the entanglement between fibers.

Regarding the analysis of the product including the fiber, for example, Japanese Patent Laying-Open No. 2016-045141 (PTL 1) discloses "A method for analyzing an image of a product including a plurality of curved fibers, the method including: acquiring a two-dimensional or three-dimensional image of the product including the plurality of curved fibers; and packing virtual particles into a fiber portion in the image, in which the fibers are analyzed as an aggregate of particles" (see [Abstract]).

### CITATION LIST

### PATENT LITERATURE

PTL 1: Japanese Patent Laying-Open No. 2016-045141

### SUMMARY OF INVENTION

### TECHNICAL PROBLEM

According to the technique disclosed in PTL 1, the structure of a single fiber cannot be estimated from the image of the product including the fiber. Accordingly, there is a need for a technique for estimating the structure of a single fiber from the image of the product including the fiber.

The present disclosure has been made in view of the above background, and an object in one aspect is to provide a technique for estimating the structure of a single fiber from the image of the product including the fiber.

### SOLUTION TO PROBLEM

A method for analyzing an image of a product including a fiber according to one embodiment includes: acquiring a three-dimensional image of the product including the fiber; transforming the fiber in the three-dimensional image into a set of particles; and estimating a structure of the fiber based on a positional relationship of each particle included in the set of particles.

In one aspect, the estimating the structure of the fiber includes generating data imitating the structure of the fiber in a region where the fiber exists based on the positional relationship of each particle included in the set of particles.

In one aspect, a set of particles is a set of points that have no radius.

In one aspect, the data imitating the structure of the fiber is one of a set of polyhedrons with a plurality of particles included, as vertices, in the set of particles, a string-like object, or a tubular object.

In an aspect, the estimating the structure of the fiber includes: increasing a radius of the particle; acquiring a first parameter of the particle when a plurality of the particles come into contact with each other to generate a cavity; obtaining a second parameter of the particle when the cavity is extinguished; and selecting the particle in a region where the fiber is presumed to exist based on the first parameter and the second parameter.

In an aspect, the selecting the particle in the region where the fiber is presumed to exist includes determining a region where the particle constituting the generated or extinguished cavity exists as the region where the fiber exists, in a case where the cavity is generated or extinguished when a radius of the particle is within a predetermined range.

In an aspect, the analysis method further includes: calculating a set of circumcenters of polyhedrons constructed with the selected particles; and estimating a position of a center line of the fiber from the set of circumcenters.

In an aspect, the analysis method further includes performing curve fitting on the set of circumcenters.

In an aspect, the analysis method further includes: calculating coordinates of a set of points evenly arranged on the center line; and estimating contacts of adjacent first fiber and second fiber based on the coordinates of the set of points.

In an aspect, the estimating the contacts of adjacent first fiber and second fiber based on the coordinates of the set of points includes: estimating a position of the circumcenter of a triangle formed by a part of the set of points in the first fiber and a part of the set of points in the second fiber; and performing curve fitting on the circumcenters of the plurality of triangles.

According to another embodiment, a program causes one or more processors to execute the above method.

According to still another embodiment, an analysis device includes: one or more processors; and a memory storing a program causing the processors to execute the above method.

### ADVANTAGEOUS EFFECTS OF INVENTION

According to one embodiment, the structure of a single fiber can be estimated from the image of the product including the fiber.

The foregoing and other objects, features, aspects and advantages of the present disclosure content will become more apparent from the following detailed description of the present disclosure when taken in conjunction with the accompanying drawings.

### BRIEF DESCRIPTION OF DRAWINGS

Fig. 1 is a view illustrating an example of an image of network structure analysis of a fiber.
Fig. 2 is a view illustrating an example of a hardware configuration of an analysis device 200 according to an embodiment.
Fig. 3 is a view illustrating an example of an outline of a method for analyzing a product including the fiber of the embodiment.
Fig. 4 is a view illustrating an example of an outline of a manufacturing process of the product including the fiber.
Fig. 5 is a view illustrating an example of conversion of the image of the product including the fibers into a point cloud.
Fig. 6 is a view illustrating an outline of persistent homology (PH) analysis processing of the point cloud.
Fig. 7 is a view illustrating an example of a procedure of plotting an analysis result of the point cloud of the fiber by a PH analysis method.
Fig. 8 is a view illustrating an example of a characteristic of the PH analysis method.
Fig. 9 is a view illustrating an example of plot data 330 obtained by performing PH analysis on the fiber converted into a point cloud 320.
Fig. 10 is a view illustrating an example of a procedure for generating data imitating a fiber structure based on the plot data 330.
Fig. 11 is a view illustrating an example of a first procedure of processing (thickening process) for estimating a contact of adjacent fibers.
Fig. 12 is a view illustrating an example of a second procedure of processing (thickening process) for estimating the contact of adjacent fibers.
Fig. 13 is a view illustrating an example of a procedure for analyzing the image of the product including the fiber.

### DESCRIPTION OF EMBODIMENT

Hereinafter, an embodiment of the technical idea according to the present disclosure will be described with reference to the drawings. In the following description, the same component is denoted by the same reference numeral. Those names and functions are the same. Accordingly, the detailed description thereof will not be repeated. The technical idea of the present disclosure is applicable to a product including a fiber and the fiber. Hereinafter, the technical idea of the present disclosure will be described using a network structure having a three-dimensional random loop junction structure formed of a continuous filament body of a thermoplastic elastomer as an example of a product including the fiber, and using the continuous filament body as an example of the fiber. The technical idea of the present disclosure is also applicable to a product including another arbitrary fiber and the another arbitrary fiber.

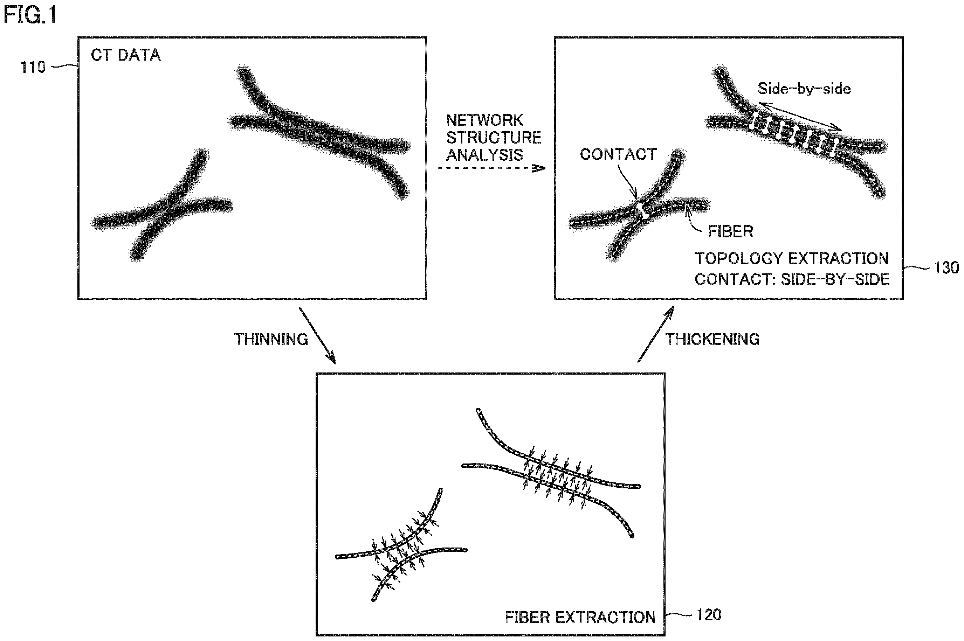

Fig. 1 is a view illustrating an example of an image of network structure analysis of a fiber. The network structure analysis of the fiber includes thinning processing and thickening processing. The thinning processing is processing for extracting the individual fiber as one line. The thinning processing reveals the structure of the individual fiber. In the example of Fig. 1, a fiber structure 120 is obtained by thinning computed tomography (CT) scan data 110. The thickening processing is processing for giving a thickness to the individual fiber obtained by the thinning processing and estimating a contact of adjacent fibers. The thickening processing reveals the contact of adjacent fibers. In the example of Fig. 1, by thickening fiber structure 120, a topology 130 including contact information about the adjacent fiber is obtained.

An analysis device of the embodiment can execute the above-described network structure analysis of the fiber, namely, the fiber thinning processing and the fiber thickening processing. A configuration of the analysis device and a flow of the network structure analysis will be described below.

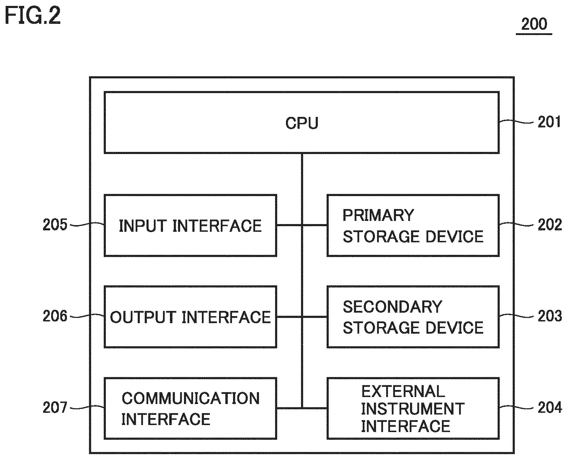

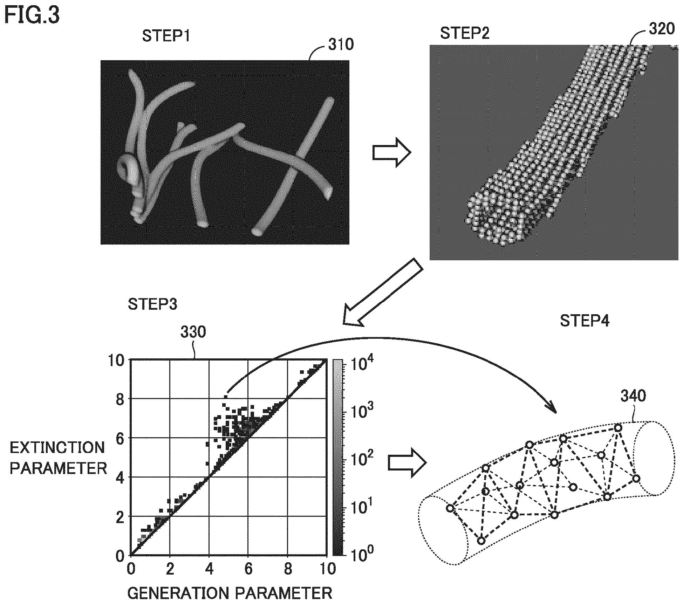

Fig. 2 is a view illustrating an example of a hardware configuration of an analysis device 200 of the embodiment. Analysis device 200 can execute a program that implements a method for analyzing an image of the product including the fiber described with reference to Fig. 3 and subsequent drawings. Analysis device 200 includes a central processing unit (CPU) 201, a primary storage device 202, a secondary storage device 203, an external instrument interface 204, an input interface 205, an output interface 206, and a communication interface 207.

CPU 201 can execute the program implementing various functions of analysis device 200. For example, CPU 201 is constructed with at least one integrated circuit. For example, the integrated circuit may include at least one CPU, at least one field programmable gate array (FPGA), or a combination thereof.

Primary storage device 202 stores the program executed by CPU 201 and data referred to by CPU 201. In one aspect, primary storage device 202 may be implemented by a dynamic random access memory (DRAM), a static random access memory (SRAM), or the like.

Secondary storage device 203 is a nonvolatile memory, and may store the program executed by CPU 201 and the data referred to by CPU 201. In this case, CPU 201 executes the program read from secondary storage device 203 to primary storage device 202, and refers to the data read from secondary storage device 203 to primary storage device 202. In one aspect, secondary storage device 203 may be implemented by a hard disk drive (HDD), a solid state drive (SSD), an erasable programmable read only memory (EPROM), an electrically erasable programmable read only memory (EEPROM), a flash memory, or the like.

External instrument interface 204 can be connected to any external device such as a printer, a scanner, and an external HDD. In one aspect, external instrument interface 204 may be implemented by a universal serial bus (USB) terminal or the like.

Input interface 205 can be connected to any input device such as a keyboard, a mouse, a touchpad, or a game pad. In one aspect, input interface 205 may be implemented by a USB terminal, a PS/2 terminal, a Bluetooth (registered trademark) module, and the like.

Output interface 206 can be connected to any output device such as a cathode ray tube display, a liquid crystal display, or an organic electro-luminescence (EL) display. In one aspect, output interface 206 may be implemented by a USB terminal, a D-sub terminal, a digital visual interface (DVI) terminal, a high-definition multimedia interface (HDMI) (registered trademark) terminal, or the like.

Communication interface 207 is connected to a wired or wireless network instrument. In one aspect, communication interface 207 may be implemented by a wired local area network (LAN) port, a wireless fidelity (Wi-Fi (registered trademark)) module, or the like. In another aspect, communication interface 207 may transmit and receive the data using a communication protocol such as a Transmission Control Protocol/Internet Protocol (TCP/IP) or a user datagram protocol (UDP).

Fig. 3 is a view illustrating an example of an outline of the method for analyzing the product including the fiber of the embodiment. With reference to Fig. 3, a series of procedures of the method for analyzing the image of the product including the fiber of the embodiment will be described. For example, the analysis method in Fig. 3 may be executed as a program by a computer having a known configuration or analysis device 200.

In step 1, analysis device 200 acquires an image 310 of the product including the fiber of an analysis target. Image 310 is a three-dimensional image of the product including the fibers of the analysis target, and can include a plurality of slice images. In one aspect, image 310 may be a CT scan image. In another aspect, image 310 may be the three-dimensional image captured using an arbitrary device such as magnetic resonance imaging (MRI).

In step 2, analysis device 200 converts each fiber in image 310 into a point cloud 320 that is a set of virtual particles. The set of the particles is a set of points having no radius. In one aspect, the set of particles may have a radius.

For example, analysis device 200 may execute conversion processing into the point cloud on each slice image in the CT scan image. In that case, analysis device 200 can generate point cloud 320 by superimposing the results of the conversion processing of each slice image into the point cloud.

In step 3, analysis device 200 performs PH analysis on a set of a plurality of particles to obtain plot data 330 as an analysis result. Details of step 3 will be described later. By referring to plot data 330, analysis device 200 can estimate a region with the fiber and a region without fiber in image 310. The analysis result obtained by the PH analysis method represents the characteristic of the distribution of each particle. Accordingly, it can also be said that analysis device 200 estimates the structure of the fiber based on a positional relationship of each particle included in the set of particles (state of distribution of each particle).

In step 4, analysis device 200 generates data 340 imitating the structure of the fiber using the analysis result by the PH analysis (by PH inverse analysis). The analysis result obtained by the PH analysis method represents the characteristic of the distribution of each particle. Therefore, it can be said that analysis device 200 generates the data imitating the structure of the fiber based on the positional relationship of each particle included in the set of particles. In one aspect, the data imitating the structure of the fiber may be data in which a set of objects such as a point or a sphere representing the particle or a polyhedron composed of the plurality of particles is disposed in the region where the fiber exists or the region of a surface portion of the fiber. In another aspect, the data imitating the structure of the fiber may be a string-like object or a tubular object including a hollow inside.

As an example, the product containing the fiber of the analysis target can include a product containing any fiber such as a network structure having a random loop structure, a nonwoven fabric, felt, paper making, acrylic, polyester, nylon, and rayon.

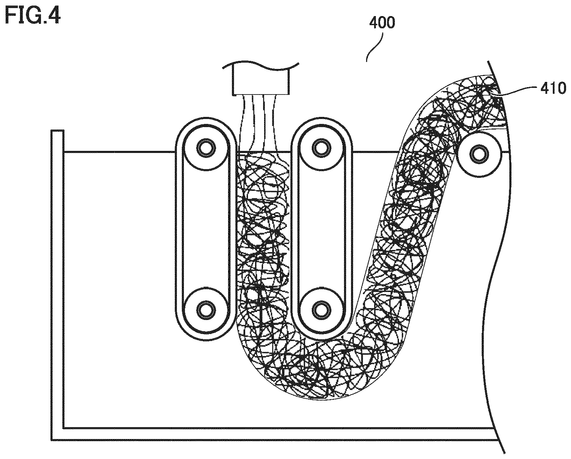

Fig. 4 is a view illustrating an example of an outline of a manufacturing process of the product including the fiber. With reference to Fig. 4 an example of use of the method for analyzing the image of the product including the fiber of the embodiment will be described. At this point, the product containing the fiber is, for example, the network structure having the three-dimensional random loop junction formed of the continuous filament body of the thermoplastic elastomer. In one aspect, the product including the fiber may be a product made of any other fiber. For example, a manufacturing device 400 for a product 410 including the fiber has a manufacturing condition of product 410 including the fiber. As an example, the manufacturing condition can include an orifice shape and a hole diameter of a nozzle that discharges resin, a distance between vertical and horizontal nozzles, a discharge temperature, a discharge amount, and an air gap (a distance from the discharge to landing on water) and a combination thereof. A person in charge of product development can manufacture product 410 including the fiber having different characteristic by changing these manufacturing conditions such that product 410 has the random loop structure required for obtaining target quality.

As an example, it is assumed that the person in charge of product development sets different manufacturing conditions for manufacturing device 400 to prototype product 410 containing the fiber twice. In this case, the person in charge of the product development can check that product 410 manufactured by any one of manufacturing conditions has the random loop structure required for obtaining the target quality by referring to the result of analyzing the three-dimensional image of each product by analysis device 200.

In one aspect, analysis device 200 may output feedback data of the analysis result of product 410 including the fiber to manufacturing device 400. In this case, manufacturing device 400 may automatically change the setting based on the feedback data, or output display prompting the person in charge of the product development to change the setting on a display or the like.

Each piece of processing in steps 1 to 4 in Fig. 3 will be described in more detail. The following processing will be described as being executed as a program by analysis device 200 as an example. With reference to Fig. 4, details of the processing for acquiring the three-dimensional image of product 410 including the fiber, which is the processing of step 1 in Fig. 3, and the processing for converting the three-dimensional image into the point cloud, which is the processing of step 2, will be described first.

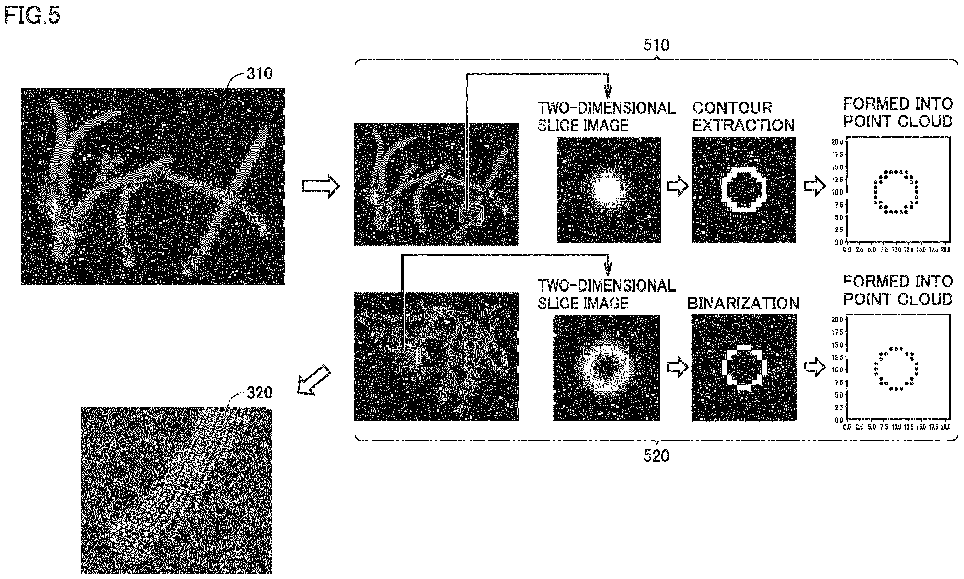

Fig. 5 illustrates an example of conversion of the image of the product including the fiber into the point cloud. In one aspect, analysis device 200 may receive image 310, which is the three-dimensional image of the product including the fiber, from a CT scanning device or the like. In another aspect, analysis device 200 may acquire image 310 through a network, a storage medium, or the like.

Analysis device 200 performs the image analysis on image 310 to generate the point cloud 320 in which the fibers are represented as a set of virtual particles. The particle may be represented as the point having no radius. In one aspect, the particle may be expressed as the sphere having the radius.

For example, analysis device 200 can analyze one of the slice images included in image 310, and arrange the particles at regular intervals in the region where the fiber is estimated to exist based on luminance information in the image. More specifically, analysis device 200 may generate point cloud 320 based on either or both of a first processing procedure 510 and a second processing procedure 520.

First processing procedure 510 is a procedure of generating point cloud 320 of a solid fiber. In first processing procedure 510, analysis device 200 acquires a two-dimensional slice image of image 310. Subsequently, analysis device 200 extracts a contour of the two-dimensional slice image. Finally, analysis device 200 generates point cloud 320 based on the contour of the two-dimensional slice image.

Second processing procedure 520 is a procedure of generating hollow-fiber point cloud 320. In second processing procedure 520, analysis device 200 acquires the two-dimensional slice image of image 310. Subsequently, analysis device 200 binarizes the two-dimensional slice image. Finally, analysis device 200 generates point cloud 320 based on the binarized two-dimensional slice image.

In both first processing procedure 510 and second processing procedure 520, analysis device 200 may generate point cloud 320 representing the void structure of the fiber. In an aspect, point cloud 320 may represent a solid structure of the fiber.

Analysis device 200 can generate the point cloud for each slice image, and finally generate point cloud 320 of the three-dimensional image by superimposing the point clouds of the respective slice images.

By arranging fine particles in units of slice images as described above, analysis device 200 can accurately arrange the particles in the region where the fiber exist even in the image in which the fibers appear to overlap such as image 310.

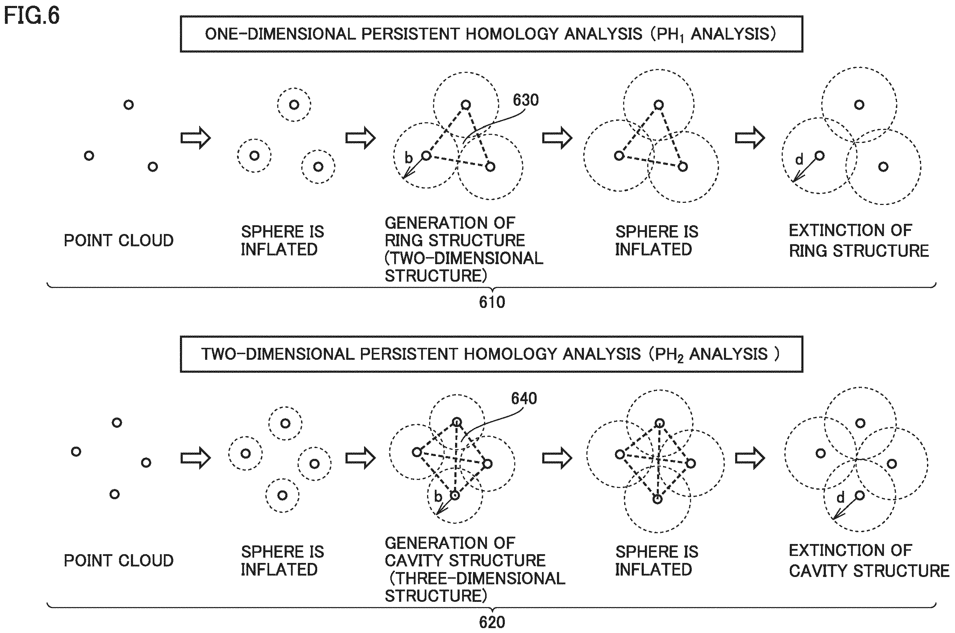

With reference to Figs. 6 to 9, details of the PH analysis processing of the point cloud, which is the processing of step 3 in Fig. 3, will be described. Fig. 6 is a view illustrating an outline of the PH analysis processing of the point cloud.

The PH analysis processing includes one-dimensional PH analysis processing (PH1 analysis) and two-dimensional analysis processing (PH2 analysis). A processing procedure 610 illustrates an example of a procedure of a one-dimensional PH analysis method. Analysis device 200 regards each particle included in the point cloud as set data of spheres (points) having no radius and gradually inflates each sphere (increases the radius of each sphere). When analysis device 200 continues to inflate each sphere, the spheres come into contact with each other at a certain timing, so that a cavity 630 (for example, a two-dimensional hole surrounded by a plurality of spheres) is generated at the center of each sphere. When analysis device 200 further continues to inflate each sphere, cavity 630 closes and extinguishes. Analysis device 200 acquires a parameter (for example, the radius of the sphere) at the generation timing (birth) and the extinction timing (death) of cavity 630. In the example of Fig. 6, the radius of each sphere at the time of the generation of cavity 630 is "b", and the radius of the sphere at the time of the extinction of cavity 630 is "d".

A processing procedure 620 illustrates an example of the procedure of the two-dimensional PH analysis method. Analysis device 200 regards each particle included in the point cloud as set data of spheres (points) having no radius and gradually inflates each sphere (increases the radius of each sphere). When analysis device 200 continues to inflate each sphere, the spheres come into contact with each other at a certain timing, so that a cavity 640 (a three-dimensional space surrounded by a plurality of spheres) is generated at the center of each sphere. When analysis device 200 further continues to inflate each sphere, cavity 640 closes and extinguishes. Analysis device 200 acquires the parameter (for example, the radius of the sphere) at the generation timing (birth) and the extinction timing (death) of cavity 640. In the example of Fig. 6, the radius of each sphere at the time of the generation of cavity 640 is "b", and the radius of the sphere at the time of the extinction of cavity 640 extinguishes is "d".

As described above, the one-dimensional PH analysis processing acquires the parameter at the generation timing and the extinction timing of the two-dimensional hole (cavity). On the other hand, the two-dimensional PH analysis processing acquires the parameter at the generation timing and the extinction timing of the three-dimensional space (cavity).

In the PH analysis method, a plurality of cavities can be generated depending on the position and number of spheres (particles). For example, it is assumed that cavities A to C are generated during the PH analysis. In this case, analysis device 200 acquires the radius of each sphere forming cavity A at the generation timing of cavity A. Similarly, analysis device 200 acquires the radius of each sphere forming cavity B at the generation timing of cavity B, and acquires the radius of each sphere forming cavity C at the generation timing of cavity C. Subsequently, it is assumed that cavities A to C disappear. In this case, analysis device 200 acquires the radius of each sphere forming cavity A until the disappearance timing of cavity A. Similarly, analysis device 200 acquires the radius of each sphere forming each of cavities B, C until the disappearance timing of cavities B, C. Then, analysis device 200 plots the radius of each sphere at the time of the generation of each of cavities A to C and the radius of each sphere at the time of extinction of each of cavities A to C in a two-dimensional coordinate.

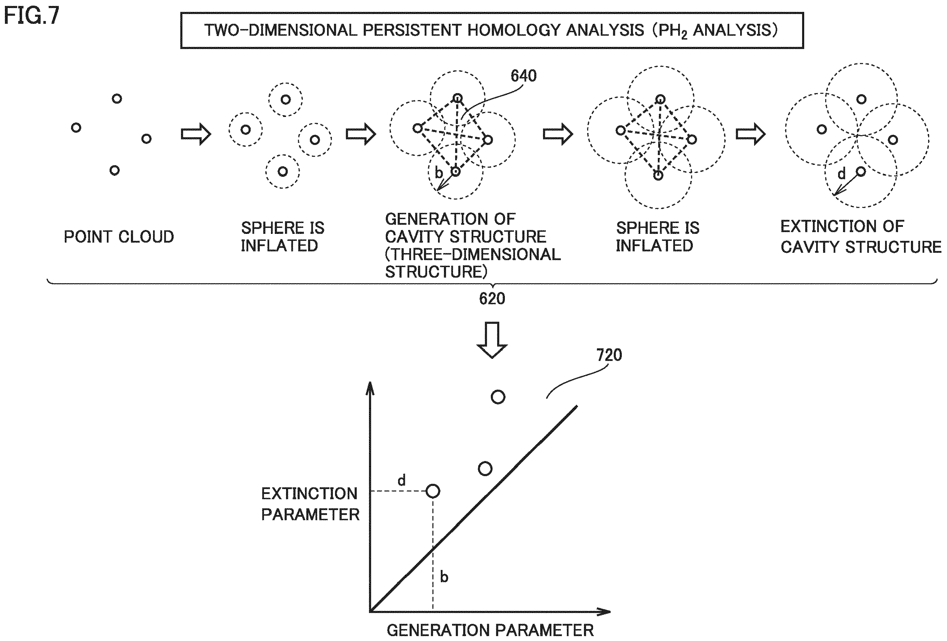

Fig. 7 is a view illustrating an example of a procedure of plotting the analysis result of the point cloud of the fiber by the PH analysis method. In plot data 720, the horizontal axis represents a generation parameter (a function represented by the radius of the sphere at the timing of the generation of the cavity), and the vertical axis represents an extinction parameter (a function represented by the radius of the sphere at the timing of the extinction of the cavity). For example, the generation and extinction parameters of cavity 640 are plotted at a coordinate P (b,d). In one aspect, the unit of the coordinate may be any value.

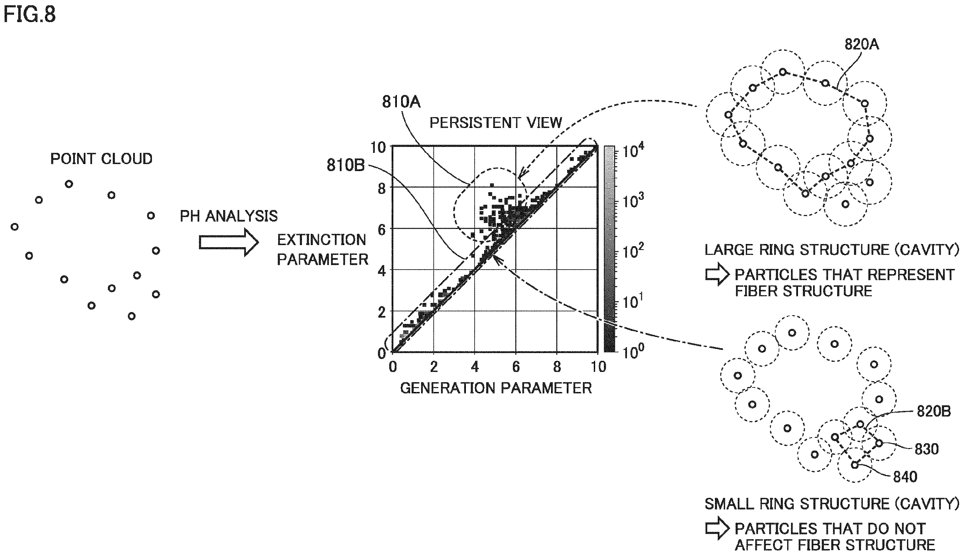

Fig. 8 is a view illustrating an example of a characteristic of the PH analysis method. The PH analysis method is characterized in that even when the position of each sphere that is the analysis target (each particle constituting the fiber converted into the point cloud) moves to some extent, the influence on the cavity is small and the method is resistant to noise. In addition, in the PH analysis method, a large ring structure 820A and a small ring structure 820B may be generated as illustrated in Fig. 8. Large ring structure 820A is included in a region 810A where the difference between the generation parameter and the extinction parameter is large. The small ring structure is included in a region 810B where the difference between the generation parameter and the extinction parameter is small. For example, analysis device 200 can exclude particles 830, 840 and the like constituting only small ring structure 820B from the point cloud as particles that do not affect the fiber structure.

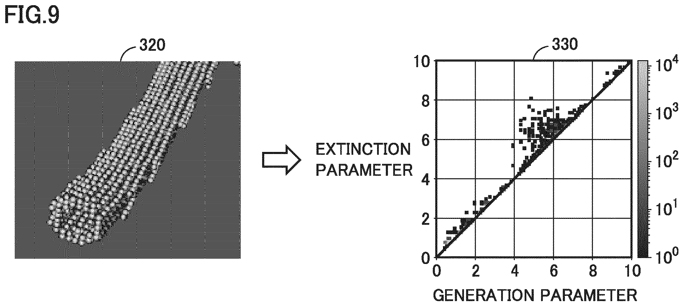

Fig. 9 is a view illustrating an example of plot data 330 obtained by performing the PH analysis on the fiber converted into point cloud 320. Analysis device 200 generates plot data 330 by performing the PH analysis on point cloud 320 based on the procedure described with reference to Figs. 6 and 7.

Analysis device 200 can estimate the structure of the fiber, the region where the fiber exists, and the like based on plot data 330. For example, it can be seen that majority of the cavities in plot data 330 is generated between generation radii "4 to 8". In this case, there is a high possibility that spheres (particles) forming the cavity at the generation radii "4 to 8" are densely arranged inside the fiber. In addition, some cavities are generated even at the generation radii greater than or equal to "8". In this case, there is a possibility that particles arranged in a vicinity of each surface of the overlapping fibers formed the cavity. For example, with reference to plot data 330, analysis device 200 can select the particle in which the generation parameter and/or the extinction parameter satisfies a specific condition, and determine the region where the selected particle exists as the region where the fiber exists.

In one aspect, analysis device 200 may select only spheres forming the cavity in which the radius of the sphere is within a predetermined range (for example, the generation radius "4 to 8"), and estimate the region including these spheres as the region where the fiber exists. In another aspect, analysis device 200 may select only the spheres forming the cavities in which the radii of the spheres are extinguished within a predetermined range (for example, the generation radius "4 to 8"), and estimate the region including these spheres as the region where the fiber exists. In another aspect, analysis device 200 may select only the spheres forming the cavities in which the radii of the spheres are generated and/or extinguished within a predetermined range (for example, the generation radii "4 to 8"), and estimate the region including these spheres as the region where the fiber exists.

That is, analysis device 200 can select the particle at the position where the fiber is estimated to exist based on the radius of the sphere when the cavity is generated and/or the radius of the sphere when the cavity is extinguished, and estimate the fiber structure and the region where the fiber exists based on the selected particle. For example, analysis device 200 can select only the particle constituting large ring structure 820A in Fig. 8.

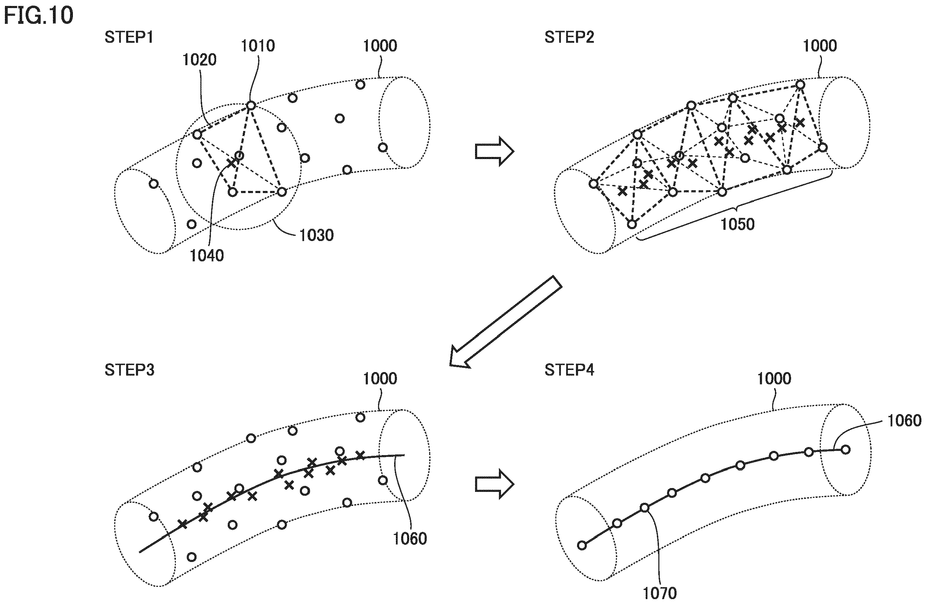

With reference to Figs. 10 to 12, processing for generating data imitating a fiber structure and processing generating topology indicating the contact of adjacent fibers, which are the processing in step 4 of Fig. 3, will be described below.

Fig. 10 is a view illustrating an example of the procedure for generating the data imitating the fiber structure based on plot data 330. In step 1, analysis device 200 analyzes plot data 330 to form a polyhedron constructed with at least one tetrahedron including a tetrahedron 1020 from a plurality of particles 1010. In one aspect, analysis device 200 may select particle 1010 existing in region 810A or the like in Fig. 8 to form the polyhedron. In other words, analysis device 200 may select particle 1010 based on the generation parameter and the extinction parameter, and form the polyhedron from selected particle 1010. In addition, analysis device 200 calculates a coordinate of a center (circumcenter) 1040 of a circumcenter circle 1030 of tetrahedron 1020.

In step 2, analysis device 200 repeatedly executes the processing of step 1 to arrange tetrahedron 1020 in the region where a fiber 1000 exists. The set of these tetrahedrons 1020 becomes data 1050 imitating the fiber structure. In addition, analysis device 200 calculates the coordinate of circumcenter 1040 of each tetrahedron 1020.

In step 3, analysis device 200 estimates the position of a center line 1060 of fiber 1000 from each circumcenter 1040. Analysis device 200 may estimate the position of the center line 1060 by performing curve fitting on each circumcenter 1040 plotted in the three-dimensional space.

In step 4, analysis device 200 obtains the coordinates of points arranged at equal intervals on center line 1060. The interval between the points can be determined arbitrarily. Analysis device 200 can estimate the contact of adjacent fibers based on these points.

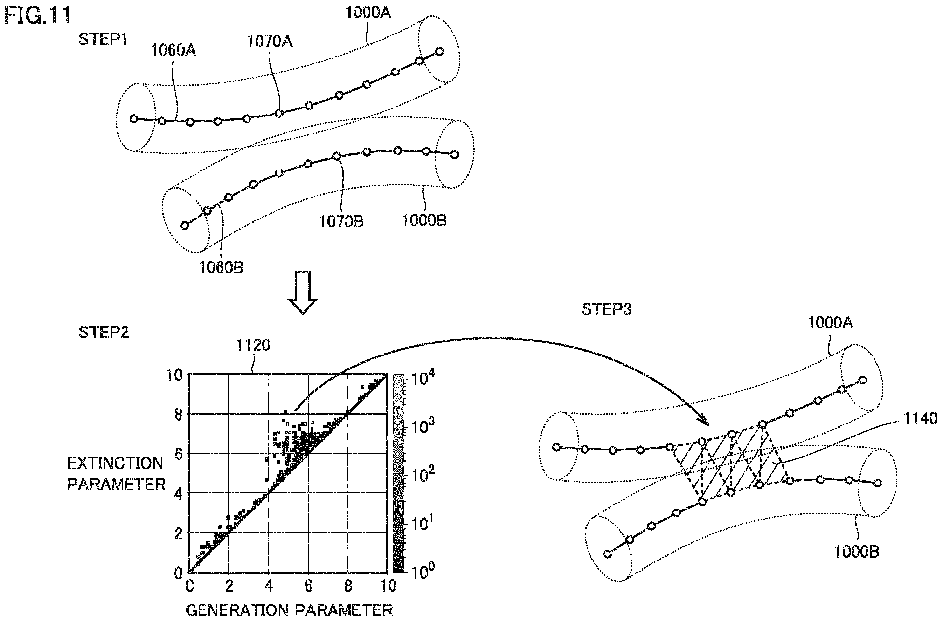

Fig. 11 is a view illustrating an example of a first procedure of processing (thickening process) for estimating the contact of adjacent fibers. In step 1, analysis device 200 calculates the coordinates of a plurality of points 1070A evenly arranged on a center line 1060A of a fiber 1000A. In addition, analysis device 200 calculates the coordinates of a plurality of points 1070B evenly arranged on a center line 1060B of a fiber 1000B.

In step 2, analysis device 200 obtains plot data 1120 by the one-dimensional PH analysis of these points. In step 3, analysis device 200 selects a point at which the generation parameter and the extinction parameter satisfy a certain condition from plot data 1120. In the example of Fig. 11, analysis device 200 selects a polygon including at least one triangle including a triangle 1140. The condition that the generation parameter and the extinction parameter are constant can be determined by the range of the generation parameter and the range of the extinction parameter. The range of each parameter can be previously determined based on an experimental result or the like.

In step 3, analysis device 200 may form a polygon constructed with at least one triangle including triangle 1140 based on the selected point. For example, in the vertices of triangle 1140, one may be selected from point 1070A of fiber 1000A and two may be selected from point 1070B of fiber 1000B. The greater the contact area of adjacent fibers 1000A, 1000B, the greater the number of triangles 1140 can be.

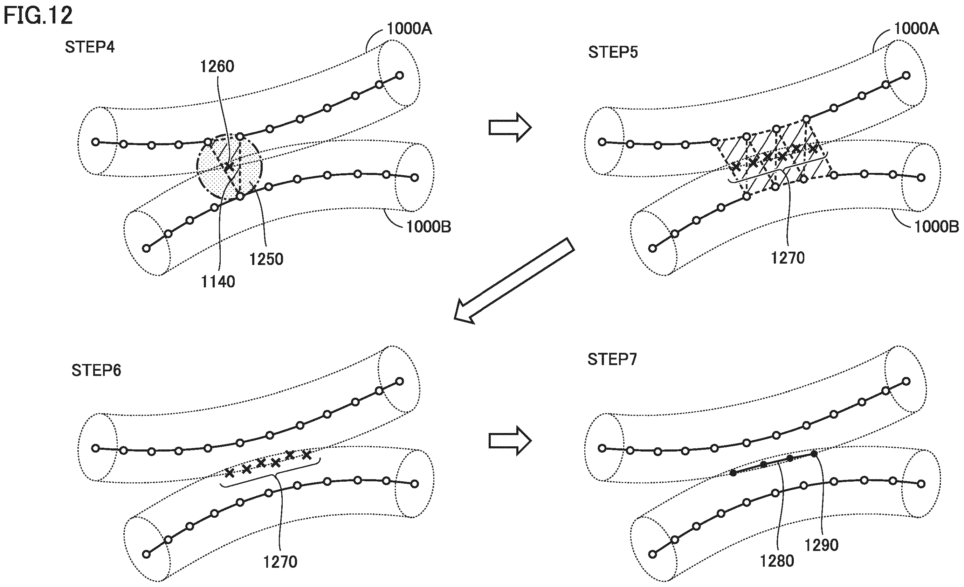

Fig. 12 is a view illustrating an example of a second procedure of processing (thickening process) for estimating the contact of adjacent fibers. The procedure of Fig. 12 is a continuation of the procedure of Fig. 11. In step 4, analysis device 200 calculates a center (circumcenter) 1260 of a circumcenter circle 1250 of triangle 1140. In step 5, analysis device 200 obtains a set of circumcenters 1270 by repeatedly executing the processing in step 4.

In step 6, analysis device 200 selects the set of circumcenters 1270. In step 7, analysis device 200 performs the curve fitting on the set of circumcenters 1270 to estimate the contact of fiber 1000A and fiber 1000B. The contacts of fiber 1000A and fiber 1000B can be represented as a curve 1280. In addition, analysis device 200 obtains the coordinates of points 1290 arranged at equal intervals on curve 1280. The interval between the points can be determined arbitrarily.

As described above, analysis device 200 can estimate the fiber structure (the center line and the set of points evenly arranged on the center line) by the processing in Fig. 10. In addition, analysis device 200 can estimate the contacts of adjacent fibers (the curve indicating the position where two fibers are in contact and the set of points evenly arranged on the curve) by the processing in Figs. 11 and 12.

For example, the person in charge of the development of product including the fiber or the like can grasp the structure of individual fibers included in the product including the fiber, the degree of entanglement between fibers, and the like by referring to information about the structure of these fibers and the contact of adjacent fibers.

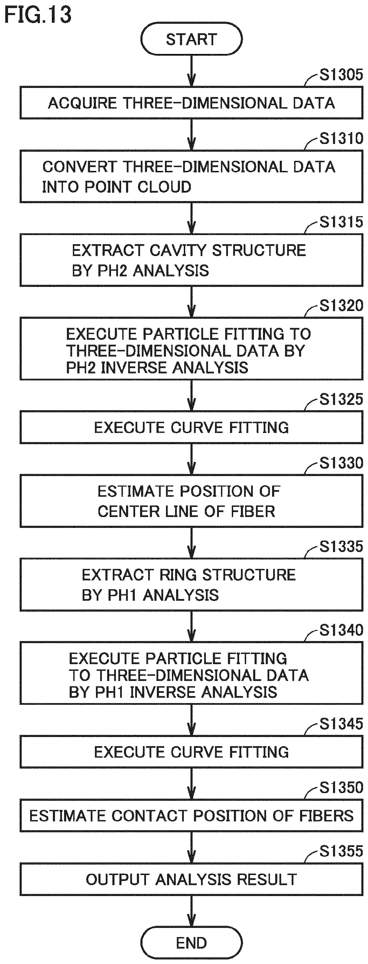

Fig. 13 is a view illustrating an example of a procedure for analyzing the image of the product including the fiber. In one aspect, CPU 201 may read the program performing the processing in Fig. 13 from secondary storage device 203 to primary storage device 202 and execute the program. In another aspect, a part or all of the processing can be implemented as a combination of circuit elements configured to execute the processing.

In step S1305, CPU 201 acquires three-dimensional image data from an external device. In one aspect, the three-dimensional image data may be a CT scan image. In another aspect, the three-dimensional image data may be three-dimensional image data captured using an arbitrary device such as MRI. In another aspect, CPU 201 may acquire the three-dimensional image data through an arbitrary network or storage medium.

In step S1310, CPU 201 converts the acquired three-dimensional image data into the point cloud. More specifically, as an example, CPU 201 analyzes two-dimensional image data that is slice data of three-dimensional image data, and produces the point cloud of each two-dimensional image data. CPU 201 can produce the point cloud of three-dimensional image data by combining the point clouds of the respective two-dimensional image data. The processing of this step corresponds to the analysis method described with reference to Fig. 5.

In step S1315, CPU 201 executes secondary PH analysis processing (PH2 analysis) on the point cloud. In addition, CPU 201 generates data in which the result of the PH2 analysis is plotted on the two-dimensional coordinate. The processing of this step corresponds to the analysis method described with reference to Figs. 6 to 9.

In step S1320, CPU 201 executes particle fitting processing to the three-dimensional data by the PH2 inverse analysis. More specifically, as an example, CPU 201 arranges the polyhedron including the plurality of (for example, four) particles included in the point cloud in the three-dimensional space, thereby generating the data imitating the fiber structure. Furthermore, CPU 201 calculates the circumcenter of each polyhedron. In one aspect, instead of arranging the polyhedron in the three-dimensional space, CPU 201 may arrange string-like data or tube-like data having a hollow inside at the position where the fiber is estimated to exist based on the analysis result of the processing in step S1315.

In step S1325, CPU 201 performs the curve fitting on the set of circumcenters obtained in step S1320. In step S1330, CPU 201 obtains the curve representing the center line of the fiber. In addition, CPU 201 calculates the coordinates of points arranged at equal intervals on the center line. The pieces of processing in steps S1320 and S1325 corresponds to the analysis method described with reference to Fig. 10.

In step S1335, CPU 201 executes the one-dimensional PH analysis (PH1 analysis) on the point set arranged on the center line calculated in step S1325, and extracts a ring structure (cavity including the plurality of points).

In step S1340, CPU 201 executes the point fitting processing to the three-dimensional data by the PH1 inverse analysis. More specifically, CPU 201 obtains the triangle including the point set arranged on the center line and the circumcenter of the triangle. In step S1345, CPU 201 performs the curve fitting on the set of the circumcenters of the triangles obtained in step S1340.

In step S1350, CPU 201 obtains the curve representing the contact of adjacent fibers. In addition, CPU 201 calculates the coordinates of the points arranged at equal intervals on the curve representing the contact of adjacent fibers. In the processing of this step, the pieces of processing in steps S1335 to S1350 corresponds to the analysis method described with reference to Figs. 11 and 12.

In step S1355, CPU 201 outputs the analysis data (data imitating the fiber structure, the center line of the fiber, information about the contact of adj acent fibers, and the like) obtained by the processing up to step S1350. In one aspect, CPU 201 may output the analysis data to the display. In another aspect, CPU 201 may transmit the analysis data to another device. In another aspect, CPU 201 may input the analysis data to another analysis program.

As described above, in the method for analyzing the image of the product including the fiber of the embodiment, the three-dimensional image data of the product including the fiber is converted into the point cloud, the data after the conversion is subjected to the PH analysis, and the data simulating the fiber structure is generated based on the analysis result. According to the analysis method, for example, the person in charge of the development of the product including the fiber or the like can analyze the individual structure of the fiber included in the manufactured product, the degree of entanglement between the fibers, and the like.

In addition, the method for analyzing the image of the product including the fiber of the embodiment estimates the fiber structure and the position of the center line based on the position of the outer center of the polyhedron including the plurality of particles constituting the point cloud. Furthermore, the analysis method can acquire the information about the contact of adj acent fibers based on the center line of the adjacent fiber. According to the analysis method, for example, the person in charge of the development of the product including the fiber or the like can grasp the degree of entanglement between fibers or the like in more detail according to the distance or the positional relationship between the center lines of the fibers.

More specifically, the person in charge of the development analyzes the shape of the fiber using the analysis technique of the image of the product including the fiber of the embodiment, thereby quantifying and evaluating the orientation of the fiber, the length of the fiber, the curvature of the fiber, the spatial position of the fiber, the density of the fiber, the distribution of pores of the fiber, or the like.

Furthermore, the person in charge of the development can quantify and evaluate the size of the contact/contact groups (side-by-side), the distribution of the contacts/contact groups, the orientation of the contact/contact groups, the position (distribution) of the contact along the fiber, the curvature between the contacts, the length between the contacts, the orientation between the contacts, or the like by analyzing the entanglement of the fibers using the method for analyzing the image of the product including the fiber of the embodiment.

It should be considered that the disclosed embodiments are an example in all respects and not restrictive. The scope of the present disclosure is defined by not the description above, but the claims, and it is intended that all modifications within the meaning and scope of the claims and their equivalents are included in the present invention. In addition, the disclosed contents described in the embodiment and the modification are intended to be implemented alone or in combination as much as possible.

### REFERENCE SIGNS LIST

110: scan data, 120: fiber structure, 130: topology, 200: analysis device, 201: CPU, 202: primary storage device, 203: secondary storage device, 204: external instrument interface, 205: input interface, 206: output interface, 207: communication interface, 310: image, 320: point cloud, 330, 720, 810, 1120: plot data, 340, 1050: data imitating fiber structure, 400: manufacturing device, 410: product, 510: first processing procedure, 520: second processing procedure, 610, 620: processing procedure, 630, 640, A, B, C: cavity, 810A, 810B: region, 820A, 820B: ring structure, 1000, 1000A, 1000B: fiber, 1010: particle, 1020: tetrahedron, 1030, 1250: circumcenter circle, 1040, 1260: circumcenter, 1070, 1290: point, 1060: center line, 1140: triangle, 1270: set of circumcenters, 1280: curve

## Claims

1. A method for analyzing an image of a product including a fiber, the method comprising:
acquiring a three-dimensional image of the product including the fiber;
transforming the fiber in the three-dimensional image into a set of particles; and
estimating a structure of the fiber based on a positional relationship of each particle included in the set of particles.

2. The analysis method according to claim 1, wherein the estimating the structure of the fiber includes generating data imitating the structure of the fiber in a region where the fiber exists based on the positional relationship of each particle included in the set of particles.

3. The analysis method according to claim 1 or 2, wherein the set of particles is a set of points having no radius.

4. The analysis method according to claim 2 or 3, wherein the data imitating the structure of the fiber is one of a set of polyhedrons with a plurality of particles included, as vertices, in the set of particles, a string-like object, or a tubular object.

5. The analysis method according to any one of claims 2 to 4, wherein
the estimating the structure of the fiber includes:
increasing a radius of the particle;
acquiring a first parameter of the particle when a plurality of the particles come into contact with each other to generate a cavity;
obtaining a second parameter of the particle when the cavity is extinguished; and
selecting the particle in a region where the fiber is presumed to exist based on the first parameter and the second parameter.

6. The analysis method according to claim 5, wherein the selecting the particle in the region where the fiber is presumed to exist includes determining a region where the particle constituting the generated or extinguished cavity exists as the region where the fiber exists, in a case where the cavity is generated or extinguished when a radius of the particle is within a predetermined range.

7. The analysis method according to claim 5 or 6, further comprising:
calculating a set of circumcenters of polyhedrons constructed with the selected particles; and
estimating a position of a center line of the fiber from the set of circumcenters.

8. The analysis method according to claim 7, further comprising performing curve fitting on the set of circumcenters.

9. The analysis method according to claim 7, further comprising:
calculating coordinates of a set of points evenly arranged on the center line; and
estimating contacts of adjacent first fiber and second fiber based on the coordinates of the set of points.

10. The analysis method according to claim 9, wherein
the estimating the contacts of adjacent first fiber and second fiber based on the coordinates of the set of points includes:
estimating a position of the circumcenter of a triangle formed by a part of the set of points in the first fiber and a part of the set of points in the second fiber; and
performing curve fitting on the circumcenters of the plurality of triangles.

11. A program causing one or more processors to execute the method of any one of claims 1 to 10.

12. An analysis device comprising:
one or more processors; and
a memory storing a program causing the processors to execute the method according to claim 11.
